# EUROPEAN PATENT APPLICATION

(11) **EP 2 609 897 A1**
(43) Date of publication of application: **03.07.2013**
(21) Application number: 11819282.2
(22) Date of filing: 21.01.2011
(51) Int. Cl.: A61F 5/44

(54) **INTELLIGENT NURSING MACHINE**

(30) Priority: 25.08.2010 CN 201010265666
(71) Applicant: Hangzhou Yinao Intelligence Technology Co. Ltd., Zhejiang 310007 (CN); Zhejiang Xinfeng Medical Apparatus Co. Ltd., Zhejiang 312368 (CN)
(72) Inventor: YING, Jiawei, Zhejiang 310007 (CN); CHEN, Hui, Zhejiang 310007 (CN); HU, Jie, Zhejiang 310007 (CN)
(74) Representative: Giles, Ashley Simon
(86) International application number: PCT/CN2011/070457
(87) International publication number: WO 2012/024907

(57) **Abstract**

This invention relates to the field of nursing machines and discloses an intelligent nursing machine including a feces collector, a connection pipe connected with the feces collector, and a sewage collector connected with the connection pipe. The feces collector includes a wearing portion and a silica gel leather cover disposed at the wearing portion. The silica gel leather cover includes at least two corrugated layers and a skin contact layer disposed at outer sides of the corrugated layers. The two adjacent corrugated layers are mutually overlaid. A feces collecting opening of the nursing machine can tightly contact the private part of the human body and does not leak the patient's feces from the lateral or rear side, greatly reducing cleaning burden on the medical personnel. The operation is very simple and convenient, suitable for the old people and patients in the hospital. The intelligent nursing machine has high economically practicality.

## Description

### BACKGROUND OF THE INVENTION

### Field of the Invention

The present invention relates to the field of nursing machines and, more particular, to an intelligent nursing machine.

### Description of the Related Art

At present, with the improvement of the living level of people, patients in bed who cannot take care of themselves because of diseases, such as cardiovascular and cerebrovascular diseases, are increasing year by year, imposing a great pressure on the family and the society. The patients need assistance from the accompanies and nursing personnel because they cannot help themselves during defecating; meanwhile, the patients suffer a lot due to irregular defecating, and in particular bear a heavy spiritual burden. The domestic patent technologies in this field are imperfect, and particularly the feces collecting openings of the traditional nursing machines cannot tightly contact the private part of the human body and easily leak feces from the lateral or rear side, which puts extra cleaning burden on the medical personnel.

### BRIEF SUMMARY OF THE INVENTION

The feces collecting opening of the nursing machine in the prior art cannot tightly contact the private part of the human body and easily leak feces from the lateral or rear side. To improve the prior art, the present invention provides a comfortable and reliable intelligent nursing machine which tightly contacts the private part of the human body and does not leak the feces from the lateral or rear side.

To solve the above technical problem, the present invention adopts the following solution:

An intelligent nursing machine includes a feces collector, a connection pipe connected with the feces collector, and a sewage collector connected with the connection pipe. The feces collector includes a wearing portion and a silica gel leather cover disposed at the wearing portion. The silica gel leather cover includes at least two corrugated layers and a skin contact layer disposed at outer sides of the corrugated layers, and the two adjacent corrugated layers are mutually overlaid.

The feces collector is worn on the private part of a user for sensing the urine and feces. The sewage collector is used for processing and storing the feces and is connected to the feces collector through the connection pipe to fulfill the aim of intelligent collection and processing of the feces. Further, the sewage collector nurses the private part of the user.

The skin contact layer is the most outside layer of the silica gel leather cover and is connected with the corrugated layers, extending flat outwardly. When a vacuum pump starts, a vacuum suction opening starts to suck air out. Under the atmospheric pressure, the folded parts of the corrugated layers of the silica gel leather cover unfold. Therefore, the most outside skin contact layer tightly contacts the skin of the private part of the user to prevent the feces from leaking. The design of the folded corrugated layers increases the elastic deformation, such that the feces collecting opening is suitable for different people with different physical shapes. Further, the laminated design of the corrugated layers prevents the silica gel leather cover from turning inwardly during use, facilitating wearing by the user.

In one embodiment of the invention, the wearing portion may include a wearing front portion and a wearing rear portion. Support side walls are disposed between the wearing front portion and the silica gel leather cover, and the support side walls are made of an elastic stretchable material. The support side walls are located at the positions where the wearing front portion contacts the inner side of the thigh of the human body, and are connected with the silica gel leather cover and the wearing front portion. The support side walls are bilaterally symmetric. The distance between the support side walls can be stretched to be fitted for different wearers with different physical shapes.

In one embodiment of the invention, the elastic stretchable material may be silica gel.

In one embodiment of the invention, the wearing front portion may be arc-shaped and may be integrally connected with the wearing rear portion. The wearing front portion and the wearing rear portion form a feces collecting opening fitted for the private part of the human body. The wearing front portion is worn on the front of the private part of the user, while the wearing rear portion is worn on the hip of the user. The two portions are connected together and are close to each other in a natural state. The connecting joint of the two portions can bear pulling and pressing to a certain extent thus to be fitted for different users with different physical shapes.

In one embodiment of the invention, an air outlet and a water outlet may be disposed inside the feces collecting opening.

In one embodiment of the invention, two liquid guide channels may be symmetrically disposed at inside walls of the feces collecting opening. The part, extending to the hip of the user, of the silica gel leather cover gradually changes from the laminated corrugated layers into a single-layer skin contact layer. Further, the liquid guide channels are formed to guide the urine to enter into the cavity of the feces collector along the liquid guide channels, preventing the urine from leaking.

In one embodiment of the invention, the liquid guide grooves may be strip-shaped grooves.

In one embodiment of the invention, the feces collector may have a cavity inside for receiving feces, and the vacuum suction opening may be disposed at the bottom of the cavity.

In one embodiment of the invention, the corrugated layers and the skin layer may be connected integrally.

In one embodiment of the invention, the corrugated layers may be stretchable folded corrugated strips.

By the above technical solution, the present invention has the technical effects as follows. The feces collecting opening of the nursing machine according to the present invention can tightly contact the private part of the user and does not leak feces from the lateral or rear side, greatly reducing cleaning burden on the medical personnel. Further, the operation is very simple and convenient, which is suitable for the old people and patients in the hospital, and the intelligent nursing machine has very high economically practicality.

### BRIEF DESCRIPTION OF THE DRAWINGS

FIG. 1 is a schematic view of the overall connection structure of the present invention;

FIG. 2 is a sectional view of a feces collector of the invention;

FIG. 3 is a three-dimensional view of the feces collector of the invention; and

FIG. 4 is a front view of FIG. 3.

Parts in the accompanying figures are as follows: feces collector 1, connection pipe 2, sewage collector, wearing portion 4, silica gel leather cover 5, corrugated layer 6, skin contact layer 7, support side wall 8, air outlet 9, water outlet 10, cavity 11, vacuum suction opening 12, front portion 41, rear portion 42, feces collecting opening 43, liquid guide channel 44.

### DETAILED DESCRIPTION OF THE INVENTION

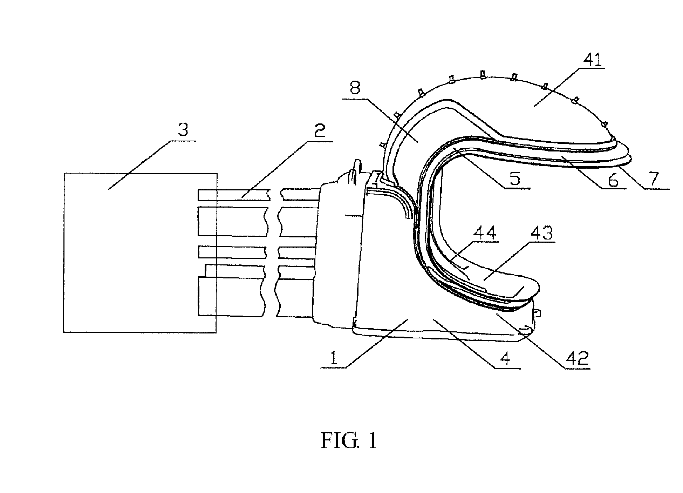

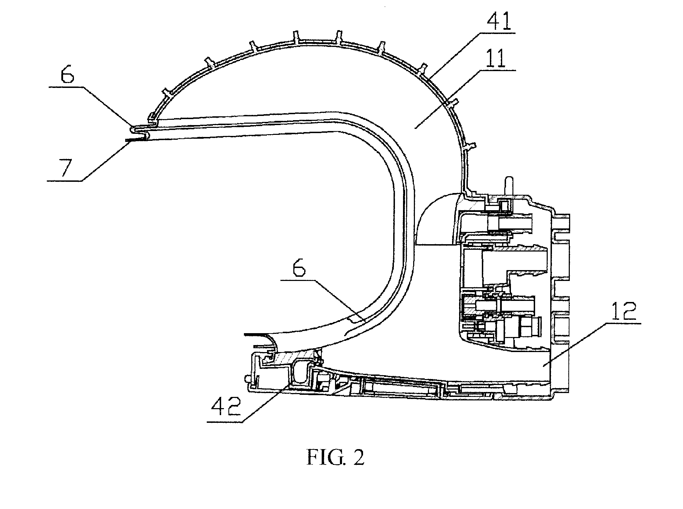

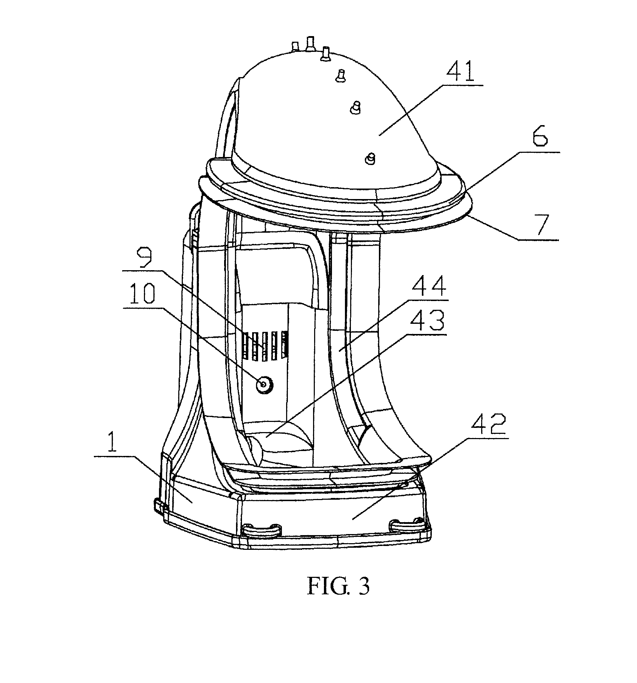

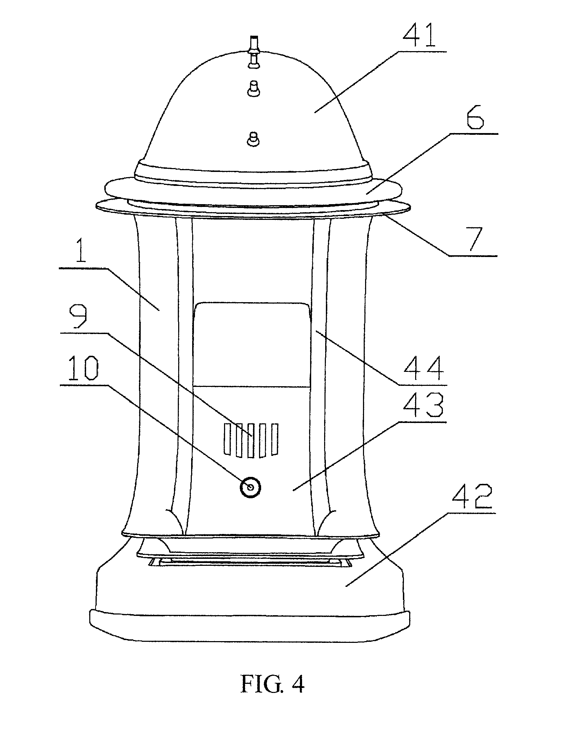

The present invention is further described in details with reference to the accompanying FIG. 1 to FIG. 4 and the specific embodiments.

As shown in FIG. 1 to FIG. 4, an intelligent nursing machine includes a feces collector 1, a connection pipe 2 connected with the feces collector 1, and a sewage collector 3 connected with the connection pipe 2. The feces collector 1 includes a wearing portion 4 and a silica gel leather cover 5 disposed at the wearing portion 4. The silica gel leather cover 5 includes at least two corrugated layers 6 and a skin contact layer 7 disposed at outer sides of the corrugated layers 6. The two adjacent corrugated layers 6 are mutually overlaid. The corrugated layers 6 and the skin contact layer 7 are integrally connected. The corrugated layers 6 are stretchable folded corrugated strips.

The feces collector 1 is worn on the private part of a user for sensing the urine and feces. The sewage collector 3 is used for processing and storing the feces and is connected to the feces collector 1 through the connection pipe 2 to fulfill the aim of intelligent collection and processing of the feces. Further, the sewage collector 3 nurses the private part of the user.

The wearing portion 4 includes a wearing front portion 41 and a wearing rear portion 42. Support side walls 8 are disposed between the wearing front portion 41 and the silica gel leather cover 5, and the support side walls 8 are made of an elastic stretchable material. The elastic stretchable material can be silica gel.

The wearing front portion 41 is arc-shaped and is integrally connected with the wearing rear portion 42. The wearing front portion 41 and the wearing rear portion 42 form a feces collecting opening 43 fitted for the private part of the human body.

An air outlet 9 and a water outlet 10 are disposed inside the feces collecting opening 43.

Two liquid guide channels 44 are symmetrically disposed at inside walls of the feces collecting opening 43. The liquid guide channels 44 can be stripe-like grooves.

The feces collector 1 has a cavity 11 inside for receiving feces, and a vacuum suction opening 12 is disposed at the bottom of the cavity 11.

The skin contact layer 7 is the most outside layer of the silica gel leather cover 5 and is connected with the corrugated layers 6, extending flat outwardly. When a vacuum pump starts, the vacuum suction opening 12 starts to suck air out. Under the atmospheric pressure, the folded parts of the corrugated layers of the silica gel leather cover 5 unfold. Therefore, the most outside skin contact layer 7 tightly contacts the skin of the private part of the user to prevent the feces from leaking. The design of the folded corrugated layers increases the elastic deformation, such that the feces colleting opening 43 is suitable for different people with different physical shapes. Further, the laminated design of the corrugated layers prevents the silica gel leather cover 5 from turning inwardly during use, facilitating wearing by the user.

The part, extending to the hip of the human body, of the silica gel leather cover 5 gradually changes from the laminated corrugated layers into the single-layer skin contact layer. Further, the liquid guide channels 44 are formed to guide the urine to enter into the cavity 11 of the feces collector 1 along the liquid guide channels 44, preventing the urine from leaking.

The support side walls 8 are located at the positions where the wearing front portion 41 contacts the inner side of the thigh of the human body, and are connected with the silica gel leather cover 5 and the wearing front portion 41. The support side walls 8 are bilaterally symmetric. The distance between the side walls 8 can be stretched to be fitted for different wearers with different physical shapes.

The wearing front portion 41 is worn on the front of the private part of the user, while the wearing rear portion 42 is worn on the hip of the user. The two portions are connected together and are close to each other in a natural state. The connecting joint thereof can bear pulling and pressing to a certain extent thus to be fitted for different users with different physical shapes.

The feces collecting opening of the nursing machine in this invention can tightly contact the private part of the human body and ensures that the feces of a patient does not leak from the lateral sides or rear side, greatly reducing cleaning burden on the medical personnel. Further, the operation is very simple and convenient, which is suitable for the old people and patients in the hospital, and the intelligent nursing machine has very high economically practicality.

To sum up, the above embodiment is only the preferable embodiment of the present invention, and all equivalent changes and modifications made on the concept of the present invention shall belong to the protective scope of the present invention.

## Claims

1. An intelligent nursing machine, comprising:
a feces collector (1);
a connection pipe (2) connected with the feces collector (1); and
a sewage collector (3) connected with the connection pipe (2), wherein the feces collector (1) includes a wearing portion (4) and a silica gel leather cover (5) disposed at the wearing portion (4), the silica gel leather cover (5) includes at least two corrugated layers (6) and a skin contact layer (7) disposed at outer sides of the corrugated layers (6), and the two adjacent corrugated layers (6) are mutually overlaid.

2. The intelligent nursing machine according to claim 1, wherein the wearing portion (4) comprises a wearing front portion (41) and a wearing rear portion (42), support side walls (8) are disposed between the wearing front portion (41) and the silica gel leather cover (5), and the support side walls (8) are made of an elastic stretchable material.

3. The intelligent nursing machine according to claim 2, wherein the elastic stretchable material is silica gel.

4. The intelligent nursing machine according to claim 2, wherein the wearing front portion (41) is arc-shaped and is integrally connected with the wearing rear portion (42), and the wearing front portion (41) and the wearing rear portion (42) form a feces collecting opening (43) fitted for the private part of the human body.

5. The intelligent nursing machine according to claim 4, wherein an air outlet (9) and a water outlet (10) is disposed inside the feces collecting opening (43).

6. The intelligent nursing machine according to claim 4 or 5, wherein two liquid guide channels (44) are symmetrically disposed at inside walls of the feces collecting opening (43).

7. The intelligent nursing machine according to claim 6, wherein the liquid guide channels (44) are stripe-shaped grooves.

8. The intelligent nursing machine according to claim 1, wherein the feces collector (1) has a cavity (11) inside for receiving feces, and a vacuum suction opening (12) is disposed at the bottom of the cavity (11).

9. The intelligent nursing machine according to claim 1, wherein the corrugated layers (6) are integrally connected with the skin contact layer (7).

10. The intelligent nursing machine according to claim 1 or 9, wherein the corrugated layers (6) are stretchable folded corrugated strips.
